# EUROPEAN PATENT APPLICATION

(11) **EP 4 088 777 A1**
(43) Date of publication of application: **16.11.2022**
(21) Application number: 21382436.0
(22) Date of filing: 11.05.2021
(51) Int. Cl.: A61N 2/00, A61N 2/02

(54) **METHOD AND SYSTEM TO TRIGGER THE ACTIVATION OF THE CORTICO-HIPPOCAMPAL MEMORY SYSTEM**

(71) Applicant: Consejo Superior de Investigaciones Científicas (CSIC), 28006 Madrid (ES)
(72) Inventor: SAN AGUSTÍN PÉREZ, Arantzazu, 28002 Madrid (ES); MORENO SASTOQUE, Juan Camilo, 28002 Madrid (ES)
(74) Representative: Hoffmann Eitle

(57) **Abstract**

In the present invention, we provide a new system and methodology aim at improving the cognitive areas responsible for memorization processes, such system and methodology is aimed at inducing synaptic plasticity toward hippocampal formation responding to a new formula to enhance the excitability of this neural network. For such purpose, in the present invention we combine a series of training tasks such as numeric-spatial memory task for activating the cortico-hippocampal memory system with a TMS pulse, preferably directed by a Neuronavigator, towards the hippocampus. Thus, one of the primary goals of the present invention is to induce plastic changes and excitability enhancement on the memory associated pathway, and a facilitation in the memory task performance

## Description

### Technical field of the invention

The present invention relates to transcranial magnetic stimulation (TMS).

### Background of the invention

The total comprehension of cognitive process of memorization remains unknown, but damage at a limited region of hippocampus is sufficient to cause easily detectable and clinically significant memory impairment in humans (Zola-Morgan et al., 1986a, Zola & Squire, 1986b). More severe declarative memory impairment is observed when the lesion includes parahipocampal gyrus, entorhinal cortex and part of perirhinal cortex (Scoville & Milner, 1957; Squire & Zola-Morgan, 1991). This type of memory refers to the ability of remembering any item and its context presented in the past. That requires an interaction between the neocortex and the medial temporal lobe memory system (Squire, 1992). Thus, the hippocampus and related structures are essential for an enduring and usable formation of declarative memories and they have become the epicentre of the memory formation research.

At cellular level, it was established synaptic plasticity as the basic mechanism of learning and memory. In 1949, Donald Hebb explained the triggering of synaptic dynamic regulation. He postulated that growth and metabolic changes occur between two neurons when two axons of different cell are close enough and repeatedly one takes part in the firing of the other (Hebb, 1949). Experience dependent reorganization of neural circuitry and novel neuronal connections are the result of synaptic efficacy regulation (Kandel, 1982). It is clear the importance of activity-dependent plasticity on synapses strength, however the temporal order of pre- and postsynaptic spiking plays a crucial role in plasticity induction too. Spike-timing-dependent plasticity (STDP) is an associative synaptic plasticity form in which the temporal synchronization of the presynaptic and postsynaptic activation determines the induction of synaptic potentiation or depression. The order and temporal inter-stimulus interval (ISI) of the neuronal action potentials will determine the directionality of the plastic changes (Buonomano, D. V., & Carvalho, T. P., 2009).

The non-invasive cortical stimulation techniques such as Transcranial Magnetic Stimulation (TMS) enabled the research of synaptic plasticity induction in human brain. Stimulation based on STDP protocol has been applied synchronizing in time and location two different nervous-system activators. This stimulation protocol called Paired Associative Stimulation (PAS) was applied primarily in primary motor cortex (M1). In 2000, Stephan et al. tested the pathway excitability changes in the motor cortical output circuitry pairing low-frequency peripheral stimulation of somatosensory afferents synchronously with TMS over the motor cortex, demonstrating reliable induction of motor cortical plasticity (Stefan et. al, 2000).

After this innovative approach of TMS application which opened the way to the possibility of inducing plasticity in a non-invasive way, several different protocols were developed combining TMS pulse with any cortex activation stimulus (San Agustín, A. and Pons, J.L., 2018 for review). In addition to synchronization with a peripheral electric pulse, TMS has been paired with interhemispheric cortico-cortical afferent projection activation induced by other TMS pulse (cc-PAS), also called Paired Bihemispheric Stimulation (PBS), producing an associative LTP-like effect (Koganemaru, 2009, Rizzo, 2008).

The synchronization of cortex activation with TMS pulse is needed for tract excitability facilitation, therefore, Thabit et al. (2010) decided to activate hand primary motor cortex in the most genuine way that motor nervous cells can be activated, asking the subjects to move hand-muscles (Thabit et al., 2010). Thus, a new protocol of PAS was designed (task-related PAS), by combining TMS cortical activation with a task that requires muscular movement 50ms before the pulse, resulting in potentiation of the corticospinal excitability and functional facilitation in task performance. In this way, they opened the door to the TMS-PAS plasticity induction in any cortex area that can be activated through a task and reached by the TMS.

### Brief description of the figures

**Figure 1****:** Selected key location for the calculation of right and left hippocampi length, hippocampi middle point depth and foot cortex depth. A. Foot motor cortex key point and corresponding surface frontal lobe scalp key point. B. Hippocampus proximal and distal key points and corresponding surface temporal lobe scalp key point.
**Figure 2****:** Horizontal, coronal and sagittal view of the selected landmarks for Neuronavigation calibration.
**Figure 3****.** Mean and Standard Error of normalized hit items of Hippocampus group and Task group during different assessment times. ^{∗} for p<0.05; ^{∗∗} for p<0.01
**Figure 4****.** Learning percentages for Hippocampus and Task group at post- and post30-assessment times.
**Figure 5****.** Mean and Standard Error of normalized hit items of low Basal Efficacy Motor Cortex group and Task group during different assessment times. ^{∗} for p<0.05; ^{∗∗} for p<0.01.
**Figure 6****.** Learning percentage respect to learning baseline (pre-assessment) for No Stimulation group (Task group) and PAS directed to foot cortex group (Motor Cortex group) in Post- and Post-30-minutes assessment.

### Description

The numerous innovative teachings of the present application will be described with particular reference to presently preferred embodiments (by way of example, and not of limitation). The present application describes several inventions, and none of the statements below should be taken as limiting the claims generally.

In the present invention, we provide a new system and methodology aim at improving the function of cognitive areas responsible for memorization processes. Such system and methodology is aimed at inducing synaptic plasticity toward hippocampal formation responding to a new formula to enhance the excitability of this neural network. For such purpose, in the present invention we combine a series of training tasks such as numeric-spatial memory task for activating the cortico-hippocampal memory system in synchronization with a TMS pulse, preferably directed by a Neuronavigator, towards the hippocampus. Thus, one of the primary goals of the present invention is to induce plastic changes and excitability enhancement on the memory associated pathway, and a facilitation in the memory task performance. In addition, the present invention further provides a system and methodology aimed at inducing synaptic plasticity toward the motor cortex combined with a series of memory training tasks such as numeric-spatial memory task for inducing plastic changes and excitability enhancement on the motor pathway, and a facilitation in the memory task performance.

It is noted that one of the main limitations of this invention was reaching the hippocampal activation by the TMS pulse. For the analysis of this possibility, previously to the invention, we compared based on RMI images the depth of a motor area that is likely to be activated by TMS and that is evaluable by its susceptibility to elicit MEPs when activated, with the depth of the hippocampus. We certify through individual RMIs that the depth of the hippocampus and motor cortex of the feet is similar intra and inter subject. Later we compare this measure with a universal atlas that we use in the PAS intervention to guide the Neuronavigator. Finally, we carried out the study of PAS with the intensity required in the motor cortex to activate the foot as a reference to apply TMS towards the hippocampus in a synchronized way with the memory task. The results are illustrated throughout the present examples.

Thus, a first aspect of the present invention refers to a system for transcranial magnetic stimulation (TMS) configured to trigger the activation of the cortico-hippocampal memory system in a subject, such as in a human patient in need thereof or in a healthy subject, wherein the system comprises:
a) an electromagnetic coil that receives power from a power source to produce a magnetic field, wherein the electromagnetic coil is configured to produce a magnetic field suitable for transcranial magnetic stimulation;
b) a processor unit (also referred to herein as controller and/or a timing control system) that controls operation of the electromagnetic coil; and
c) optionally, an interface that includes the electromagnetic coil, the interface configured to position the electromagnetic coil to deliver controlled transcranial magnetic stimulation (TMS) pulses to the hippocampus of the patient during a stimulation period,
wherein the processor unit that controls operation of the electromagnetic coil is further configured, via an operating protocol preferably store in a memory configured to store said operating protocol, to provide instructions to a subject connected to the system through a terminal that displays training actions such as memory tasks to the subject, according to the operating protocol, wherein said operating protocol is configured to repeatedly, randomly or subsequently, performing one or more training actions such as memory tasks, wherein at least one of the repeated performances is temporally linked with the delivery of transcranial magnetic stimulation (TMS) pulses to the hippocampus of the subject during a stimulation period.

In a preferred embodiment of the first aspect of the invention, the system of the first aspect of the invention may not necessarily comprise an interface that houses the coil, and the coil may be thus placed in the subject scalp to direct the magnetic stimulation to the hippocampal formation by any other means such as by the operator of the system. However, in a preferred embodiment of the first aspect of the invention, the electromagnetic coil is housed in an interface that facilitates quick and easy positioning of the coil proximate to a subject's brain. A controller is provided that runs the electromagnetic coil according to a defined, operating protocol that further simplifies the use of the device. An interface may incorporate one or more electromagnetic coils such that, during use, the coil(s) are positioned to deliver a magnetic field. According to an embodiment of the invention, the interface includes a pliable hat-shaped device that can be fitted rapidly to most any subject's head. Alternately, the interface may comprise a pliable envelope with flexible, resilient bands that, when worn by a subject, extend downward from the subject's crown toward the subject's hairline. These bands may be expanded so that the interface can slide over a subject's head, positioning the electromagnetic coil(s) to deliver a magnetic field to the subject's brain.

In another preferred embodiment of the first aspect of the invention or of any of its preferred embodiments, the system of the first aspect of the invention may comprise a Neuronavigator to assist the coil placement in the subject scalp in order to direct the magnetic stimulation to the hippocampal formation. In another preferred embodiment of the first aspect of the invention or of any of its preferred embodiments, the system of the first aspect of the invention may optionally further comprise a plurality of electrodes configured to receive a signal indicative of brain activity in the subject and to provide the electrical signal to the processor unit. It is noted that the plurality of electrodes can be used for determining the intensity required in the motor cortex to activate the foot as a reference to apply TMS towards the hippocampus in a synchronized way with the memory task.

A second aspect of the invention refers to a system fortranscranial magnetic stimulation (TMS) configured to trigger the activation of the motor cortex in a subject, such as in a human patient in need thereof or in a healthy subject, wherein the system comprises:
a) an electromagnetic coil that receives power from a power source to produce a magnetic field, wherein the electromagnetic coil is configured to produce a magnetic field suitable for transcranial magnetic stimulation;
b) a processor unit (also referred to herein as controller and/or a timing control system) that controls operation of the electromagnetic coil; and
c) optionally, an interface that includes the electromagnetic coil, the interface configured to position the electromagnetic coil to deliver controlled transcranial magnetic stimulation (TMS) pulses to the motor cortex of the patient during a stimulation period,
wherein the processor unit that controls operation of the electromagnetic coil is further configured, via an operating protocol preferably store in a memory configured to store said operating protocol, to provide instructions to a subject connected to the system through a terminal that displays memory tasks to the subject, according to the operating protocol, wherein said operating protocol is configured to repeatedly, randomly or subsequently, performing one or more memory tasks, wherein at least one of the repeated performances is temporally linked with the delivery of transcranial magnetic stimulation (TMS) pulses to the motor cortex of the subject during a stimulation period.

As indicated in the examples of the present specification, the system of the second aspect of the invention is especially suitable for activating the spatial memory, that is, the memory responsible for the storage and retrieval of information within the brain that is needed both to plan a route to a desired location and to remember where an object is located or where an event occurred.

In a preferred embodiment of the second aspect of the invention, the system of the second aspect of the invention may not necessarily comprise an interface that houses the coil, and the coil may be thus placed in the subject scalp to direct the magnetic stimulation to the hippocampal formation by any other means such as by the operator of the system. However, a preferred embodiment of the second aspect of the invention, the electromagnetic coil is thus housed in an interface that facilitates quick and easy positioning of the coil proximate to a subject's brain. A controller is provided that runs the electromagnetic coil according to a defined, operating protocol that further simplifies the use of the device. An interface may incorporate one or more electromagnetic coils such that, during use, the coil(s) are positioned to deliver a magnetic field. According to an embodiment of the invention, the interface includes a pliable hat-shaped device that can be fitted rapidly to most any subject's head. Alternately, the interface may comprise a pliable envelope with flexible, resilient bands that, when worn by a subject, extend downward from the subject's crown toward the subject's hairline. These bands may be expanded so that the interface can slide over a subject's head, positioning the electromagnetic coil(s) to deliver a magnetic field to the subject's brain.

In another preferred embodiment of the second aspect of the invention or of any of its preferred embodiments, the system of the second aspect of the invention may comprise a Neuronavigator to assist the coil placement in the subject scalp to direct the magnetic stimulation to the motor cortex. In another preferred embodiment of the second aspect of the invention or of any of its preferred embodiments, the system of the second aspect of the invention may optionally further comprise a plurality of electrodes configured to receive a signal indicative of brain activity in the subject and to provide the electrical signal to the processor unit.

It is noted that, in the context of the present invention, by repeatedly performing one or more training actions such as memory tasks is meant that the training system displays at least one training action such as memory tasks to the patient, according to an operating protocol, wherein if more than one training action is performed such training actions are display, randomly or subsequently, each occurring at different times, that is, there is preferably no overlap in the timing of display of each of the training action. In some embodiments, a training action may be immediately followed by a further training action while in other embodiments, a training action may be followed by a further training action 1, 2, 3, 4, 5, 6, 10, 20, 30 minutes or more later.

It is further noted that, the system, of the first or second aspect of the invention, should further comprise, preferably a data storage system to store the operating protocol and training actions, and a terminal display for the subject to visualize the training actions such as the memory tasks. Such display can be replaced by any other manner of presenting the training actions to the patient, such as any suitable interacting means with the patient. The system should in addition provide means, preferably input means, that credibly assists the patient in performing the training actions by means of a continued and/or guided human-machine interaction process. For example, the system can display to the subject, as training actions in the form of memory tasks, several numbers presented in a random arrangement within a button matrix; providing a retention phase, where the elements disappear, but leave a blank track; and a recovery phase in which the subject has to press the buttons following the numerical order of the items. Therefore, the system should further comprise, preferably a data storage system, a display for the subject to visualize the training actions such as the memory tasks and means that assists the subject in performing the training actions such as memory tasks by means of a continued and/or guided human-machine interaction process.

It is further noted that, in the context of the present invention, the subject is any human being in need to improve at least one of concentration, memory, or cognitive performance. The subject may be diagnosed with ADHD (Attention deficit hyperactivity disorder) or may be suffering from age-related memory loss or be at risk for age-related memory loss or may be suffering from any cognitive disorders including Dementia, Developmental disorders, Motor skill disorders, Amnesia, Substance-induced cognitive impairment, Alzheimer's disease, Behavioral variant frontotemporal dementia. Corticobasal degeneration, Huntington's disease, Lewy body dementia (or dementia with Lewy bodies), Mild cognitive impairment, Primary progressive aphasia, or Progressive supranuclear palsy. The subject may have significant difficulty focusing or concentrating. The subject may have developmental impairment of executive function. The subject may need treatment to improve cognitive performance in areas including but not limited to, standardized or other educational tasks or testing or occupational tasks or testing.

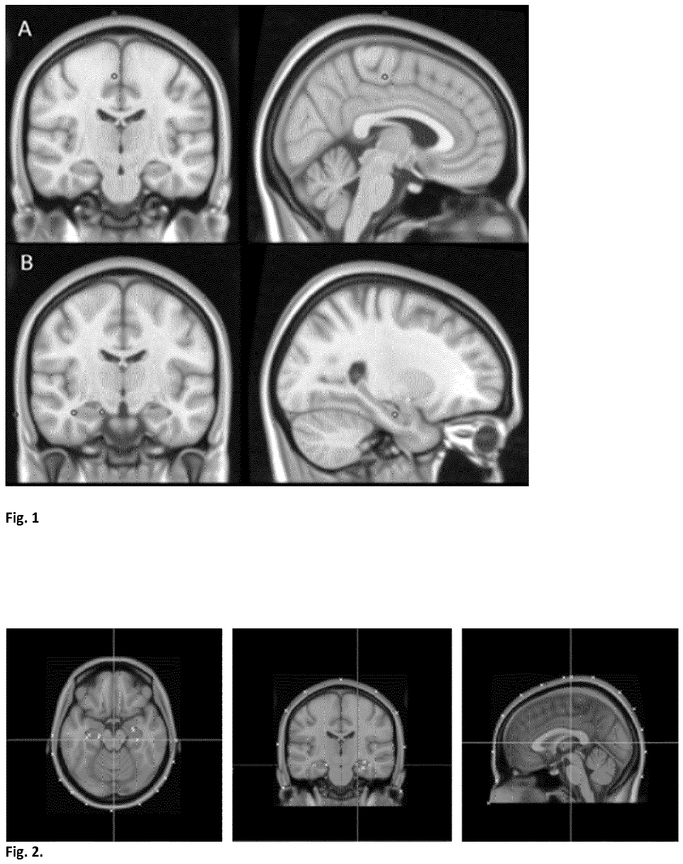

It is still further noted that, as already stated, to assist the positioning the electromagnetic coil to deliver controlled transcranial magnetic stimulation (TMS) pulses, in particular to the hippocampus of the patient, during a stimulation period, a frameless stereotactic Brainsight TMS Navigation can be preferably used. Such system can assist the coil placement in the subject scalp to direct the magnetic stimulation to hippocampal formation. This system is generally based on an optical (infrared) position sensor NDI Polaris camera (Vicra / Spectra) tracking method. In this sense, the participant's head is aligned with the TMS coil position in 3D real space to MNI152 image space in the screen. Firstly, the central point of the coil that will be in contact with the scalp is calibrated with the trackers placed in the handle of the coil. Thus, the TMS central point in the screen space can be identified. Then, a number of anatomical landmarks can be determined in the MRI image (Figure 2) that can be identify in subject's head (physical space coordinates), locating trackers on a tape that is placed around the subject's head and using a tracked pointer to indicate physically the designated landmarks in subject head and face. This way, it can be visualized in real time from physical space to image space, the center point of TMS coil location in relation to the head and brain structures, enabling the precise placement in the scalp to direct the stimulation, in particular to the hippocampus.

Thus, with reference to the first or second aspect of the invention, a paired training system is shown, wherein the processor unit, controller and/or a timing control system is communicably connected to the electromagnetic coil and to a terminal that displays training actions such as memory tasks to the patient, according to an operating protocol. Receiving timing instruction from the controller and/or a timing control system, the electromagnetic coil provides stimulation to the hippocampus or to the motor cortex (depending on whether the system is in accordance with the first or second aspect of the invention). Similarly receiving timing instruction from the controller and/or a timing control system or providing timing instruction to the controller and/or a timing control system, the terminal displays the desired memory tasks that the subject will preferably visualize. The result of these tasks should preferably reflect short-term memory, it should be as simple as possible to rule out external factors. Moreover, it is important that each cycle of memory tasks has a coding phase and a recall phase, in order to apply the TMS in each trial. The memory performance should preferably be not only recognition, but an exercise of active memory, to be able to give results limited to the capacity of memorization and not of recognition. Preferably, this task has a coding phase in which numbers are presented in a random arrangement within a button matrix; a retention phase, where the elements disappear, but leave a blank track; and a recovery phase in which the subject has to press the buttons following the numerical order of the items.

The TMS PAS of present intervention is thus based in the convergence of two stimuli in the same neuronal population. In this sense, the present invention converges the TMS pulse with the genuine activation of hippocampal formation in the medial temporal lobe of the brain by the memorization task or in the motor cortex (depending on whether the system is in accordance with the first or second aspect of the invention). Thus, the TMS is applied, preferably in a single pulse, synchronized with the encoding phase of the memorization task, when the items to memorize are presented.

The controller and/or a timing control system generally provides the simultaneous nature of the pairing. The stimulation and the training when simultaneously produced, are meant to be simultaneous in that they occur at the same time, that is, there is at least some overlap in the timing, preferably, there is a parallel communication. In some embodiments of the first or second aspect, the stimulation may lead the start of the training while in other embodiments, the stimulation may follow the start of the training. In many cases, the stimulation is shorter in duration than the training, such that the stimulation occurs near the beginning of the training. Plasticity resulting from stimulation has been shown to last minutes or hours, so a single stimulation pulse may suffice for the whole duration of extended training. In preferred embodiments, the system is thus configured to trigger the delivery of the transcranial magnetic stimulation (TMS) pulses to the hippocampus or to the motor cortex (depending on whether the system is in accordance with the first or second aspect of the invention) of the subject during a stimulation period simultaneously to commencing the display of the memory tasks to the patient.

In another preferred embodiment, the system, of the first or second aspects of the invention, further comprises an initiated timing control system. The initiated timing control system includes an initiated timing control. The initiated timing control is communicably connected to the coil and an event generator. The initiated timing control receives timing information from the electrodes of the system, indicating that the hippocampus or the motor cortex (depending on whether the system is in accordance with the first or second aspect of the invention) has been stimulated. The initiated timing control then provides timing instructions to the terminal, such that the terminal displays the memory task during the stimulation pulse.

In another preferred embodiment, the system, of the first or second aspects of the invention, may comprise a delayed response timing control system. The delayed response timing control system includes a delayed response timing control. The delayed response timing control is communicably connected to coil and a preliminary event sensor. The preliminary event sensor detects a preliminary event that anticipates a pairing event. The delayed response timing control receives timing information from the preliminary event sensor, indicating that a preliminary event has been detected. The delay response timing control provides timing instructions to the coil to initiate the hippocampus or the motor cortex (depending on whether the system is in accordance with the first or second aspect of the invention) stimulation. In this embodiment, the timing control initiates the stimulation before the beginning of the pairing event, giving a negative delta t. A delay response timing system may initiate stimulation at the same time as the beginning of the pairing event, or after the beginning of the pairing event.

In a preferred embodiment of the first or second aspect of the invention, the electromagnetic coil is configured to deliver a single pulse of transcranial magnetic stimulation (TMS) to the hippocampus or to the motor cortex (depending on whether the system is in accordance with the first or second aspect of the invention) of the *subject* during the stimulation period. Preferably, single pulse of TMS is apply at an intensity in the range of from about 80% to 160% of the Resting Motor Threshold (RMT) of the foot motor cortex to the hippocampus, wherein the RMT is the stimulation intensity that evokes 5 out of 10 times a Motor Evoked Potential (MEP) with a peak-to-peak amplitude of 50µV while the muscle is at a resting state. More preferably, single pulse of TMS is applied at an intensity in the range of from about 100% to 140% of the Resting Motor Threshold (RMT) of the foot motor cortex to the hippocampus. Still more preferably, a single pulse of TMS is apply at an intensity of about 120% of the Resting Motor Threshold (RMT) of the foot motor cortex to the hippocampus.

In another preferred embodiment of the first or second aspect of the invention or of any of its preferred embodiments, the system further comprises means, such as an optical position sensor, to assist the coil placement in the patient scalp to direct the magnetic stimulation to the hippocampus or to the motor cortex (depending on whether the system is in accordance with the first or second aspect of the invention).

In another preferred embodiment of the first or second aspect of the invention or of any of its preferred embodiments, the training actions are memory tasks to be performed by the patient, having a coding phase, a retention phase and a recovery phase.

In another preferred embodiment of the first or second aspect of the invention or of any of its preferred embodiments, the system is configured to in addition to at least one of the repeated training actions being temporally linked with the delivery of transcranial magnetic stimulation (TMS) pulses to the hippocampus or to the motor cortex (depending on whether the system is in accordance with the first or second aspect of the invention) of the subject during a stimulation period, said training actions are repeated before the stimulation period without being temporally linked with the delivery of transcranial magnetic stimulation (TMS) pulses.

In another preferred embodiment of the first or second aspect of the invention or of any of its preferred embodiments, the system is configured to in addition to at least one of the repeated training actions being temporally linked with the delivery of transcranial magnetic stimulation (TMS) pulses to the hippocampus or to the motor cortex (depending on whether the system is in accordance with the first or second aspect of the invention) of the subject during a stimulation period, said training actions are repeated one or more times after the stimulation period without being temporally linked with the delivery of transcranial magnetic stimulation (TMS) pulses.

In still another preferred embodiment of the first or second aspect of the invention or of any of its preferred embodiments, the system, and in particular the processor unit, is configured to repeatedly display at least four training actions, in particular a pre-assessment: Evaluation of the basal capacity of memorization of the patient through a learning or memory task, a PAS intervention: stimulation application temporally linking the delivery of a single pulse of TMS towards the hippocampus or to the motor cortex (depending on whether the system is in accordance with the first or second aspect of the invention) with endogenous activation of the hippocampal pathway or to the motor cortex due to the completion of the task, Post-assessment: Evaluation of the ability to memorize by performing the same task immediately after the intervention, and an optional second Post-assessment: evaluation of the memorization capacity 1, 2, 3, 4, 5, 10, 20 or preferably 30 minutes after the intervention.

A third aspect of the invention refers to a method carried out by a computer to trigger the activation of the cortico-hippocampal memory system in a subject in need thereof by using a neural stimulator comprising:
1. an electromagnetic coil that receives power from a power source to produce a magnetic field, wherein the electromagnetic coil is configured to produce a magnetic field suitable for transcranial magnetic stimulation;
2. a processor unit (also referred to herein as controller and/or a timing control system) that controls operation of the electromagnetic coil; and
3. optionally, an interface that includes the electromagnetic coil, the interface configured to position the electromagnetic coil to deliver controlled transcranial magnetic stimulation (TMS) pulses to the hippocampus of the patient during a stimulation period,
wherein the processor unit that controls operation of the electromagnetic coil is further configured, via an operating protocol preferably store in a memory configured to store said operating protocol, to provide instructions to a subject connected to the neural stimulator through a terminal that displays training actions such as memory tasks to the subject, according to the operating protocol, wherein the method comprises, in accordance with the operating protocol, to repeatedly performing one or more training actions such as memory tasks, wherein at least one of the repeated performances is temporally linked with the delivery of transcranial magnetic stimulation (TMS) pulses to the hippocampus of the subject during a stimulation period.

As reflected in the first aspect of the invention, in a preferred embodiment of the third aspect of the invention, the electromagnetic coil may be optionally housed in an interface that facilitates quick and easy positioning of the coil proximate to a subject's brain. An interface may incorporate one or more electromagnetic coils such that, during use, the coil(s) are positioned to deliver a magnetic field. According to an embodiment of the invention, the interface includes a pliable hat-shaped device that can be fitted rapidly to most any subject's head. Alternately, the interface may comprise a pliable envelope with flexible, resilient bands that, when worn by a subject, extend downward from the subject's crown toward the subject's hairline. These bands may be expanded so that the interface can slide over a subject's head, positioning the electromagnetic coil(s) to deliver a magnetic field to the subject's brain.

In another preferred embodiment of the third aspect of the invention or of any of its preferred embodiments, the system of the third aspect of the invention may comprise a Neuronavigator to assist the coil placement in the subject scalp in order to direct the magnetic stimulation to the hippocampal formation. In another preferred embodiment of the third aspect of the invention or of any of its preferred embodiments, the system of the third aspect of the invention may optionally further comprise a plurality of electrodes configured to receive a signal indicative of brain activity in the subject and to provide the electrical signal to the processor unit. It is noted that the plurality of electrodes can be used for determining the intensity required in the motor cortex to activate the foot as a reference to apply TMS towards the hippocampus in a synchronized way with the memory task.

A fourth aspect of the invention refers to a method carried out by a computer to trigger the activation of the memory system via the activation of the motor cortex in a subject in need thereof by using a neural stimulator comprising:
1. an electromagnetic coil that receives power from a power source to produce a magnetic field, wherein the electromagnetic coil is configured to produce a magnetic field suitable for transcranial magnetic stimulation;
2. a processor unit (also referred to herein as controller and/or a timing control system) that controls operation of the electromagnetic coil; and
3. an interface that includes the electromagnetic coil, the interface configured to position the electromagnetic coil to deliver controlled transcranial magnetic stimulation (TMS) pulses to the hippocampus of the patient during a stimulation period,
wherein the processor unit that controls operation of the electromagnetic coil is further configured, via an operating protocol preferably store in a memory configured to store said operating protocol, to provide instructions to a subject connected to the neural stimulator through a terminal that displays memory tasks to the subject, according to the operating protocol, wherein the method comprises, in accordance with the operating protocol, to repeatedly performing one or more memory tasks, wherein at least one of the repeated performances is temporally linked with the delivery of transcranial magnetic stimulation (TMS) pulses to the motor cortex of the subject during a stimulation period.

As reflected in the second aspect of the invention, in a preferred embodiment of the fourth aspect of the invention, the electromagnetic coil may be optimally housed in an interface that facilitates quick and easy positioning of the coil proximate to a subject's brain. An interface may incorporate one or more electromagnetic coils such that, during use, the coil(s) are positioned to deliver a magnetic field. According to an embodiment of the invention, the interface includes a pliable hat-shaped device that can be fitted rapidly to most any subject's head. Alternately, the interface may comprise a pliable envelope with flexible, resilient bands that, when worn by a subject, extend downward from the subject's crown toward the subject's hairline. These bands may be expanded so that the interface can slide over a subject's head, positioning the electromagnetic coil(s) to deliver a magnetic field to the subject's brain.

In another preferred embodiment of the fourth aspect of the invention or of any of its preferred embodiments, the system of the fourth aspect of the invention may comprise a Neuronavigator to assist the coil placement in the subject scalp in order to direct the magnetic stimulation to the motor cortex. In another preferred embodiment of the fourth aspect of the invention or of any of its preferred embodiments, the system of the fourth aspect of the invention may optionally further comprise a plurality of electrodes configured to receive a signal indicative of brain activity in the subject and to provide the electrical signal to the processor unit. It is noted that the plurality of electrodes can be used for determining the intensity required in the motor cortex to activate the foot as a reference to apply TMS towards the hippocampus in a synchronized way with the memory task.

It is noted that although transcranial magnetic stimulation is much preferred for the method of the present invention, neural stimulation can be achieved by any other system which may provide stimulation of the hippocampus or to the motor cortex such as by using electrical stimulation, chemical stimulation, magnetic stimulation, optical stimulation, mechanical stimulation or any other form of suitable stimulation. In accordance with an embodiment, an electrical stimulation is provided to the hippocampus or to the motor cortex. In an electrical stimulation system, suitable stimulation pulses may include a variety of waveforms, including constant current pulses, constant voltage pulses, exponential pulses or any other appropriate waveform. An electrical stimulation system may use a single stimulation pulse or a train of stimulation pulses to stimulate the hippocampus or to the motor cortex. Stimulation parameters are selected to affect the brain appropriately, with reference to the affected brain regions or systems, plasticity measures, desynchronization or any other appropriate stimulation parameter measure.

Paired stimulation is, however, much preferably accomplished using transcranial magnetic stimulation.

It is further noted that, the method could further comprise, preferably a data storage system to store the operating protocol and training actions, and a display for the subject to visualize the training actions such as the memory tasks. Such display can be replaced by any other manner of presenting the training actions to the patient, such as any suitable interacting means with the subject. The method should in addition provide means, preferably input means, that credibly assists the subject in performing the training actions by means of a continued and/or guided human-machine interaction process.

Thus, with reference to the third or fourth aspect of the invention, a method is shown, wherein the controller and/or a timing control system (processor unit) is communicably connected to the electromagnetic coil and to a terminal that displays training actions such as memory tasks to the subject, according to an operating protocol. Receiving timing instruction from the controller and/or a timing control system, the electromagnetic coil provides stimulation to the hippocampus or to the motor cortex (depending on whether the method is in accordance with the third or fourth aspect of the invention). Similarly receiving timing instruction from the controller and/or a timing control system or providing timing instruction to the controller and/or a timing control system, the terminal displays the desired memory tasks that the subject will preferably visualize. The result of these tasks causes the convergence of two stimuli in the same neuronal population. In this sense, the methodology of the present invention converges the TMS pulse with the genuine activation of hippocampal formation in the medial temporal lobe of the brain or in the motor cortex by the memorization task. Thus, the TMS is applied, preferably in a single pulse, synchronized with the encoding phase of the memorization task, when the items to memorize are presented.

The controller and/or a timing control system generally provides the simultaneous nature of the pairing. The stimulation and the training when simultaneously produced, are meant to be simultaneous in that they occur at the same time, that is, there is at least some overlap in the timing. In some embodiments, the stimulation may lead the start of the training while in other embodiments, the stimulation may follow the start of the training. In many cases, the stimulation is shorter in duration than the training, such that the stimulation occurs near the beginning of the training. Plasticity resulting from stimulation has been shown to last minutes or hours, so a single stimulation pulse may suffice for the whole duration of extended training. In preferred embodiments, the system is thus configured to trigger the delivery of the transcranial magnetic stimulation (TMS) pulses to the hippocampus or to the motor cortex (depending on whether the method is in accordance with the third or fourth aspect of the invention) of the patient during a stimulation period simultaneously to commencing the display of the memory tasks to the patient.

In another preferred embodiment, the method further comprises an initiated timing control system. The initiated timing control system includes an initiated timing control. The initiated timing control is communicably connected to the coil and an event generator. The initiated timing control receives timing information from the electrodes of the system, indicating that the hippocampus or the motor cortex (depending on whether the method is in accordance with the third or fourth aspect of the invention) has been stimulated. The initiated timing control then provides timing instructions to the terminal, such that the terminal displays the memory task during the stimulation pulse.

In another preferred embodiment, the method may comprise a delayed response timing control system. The delayed response timing control system includes a delayed response timing control. The delayed response timing control is communicably connected to coil and a preliminary event sensor. The preliminary event sensor detects a preliminary event that anticipates a pairing event. The delayed response timing control receives timing information from the preliminary event sensor, indicating that a preliminary event has been detected. The delay response timing control provides timing instructions to the coil to initiate the hippocampus or the motor cortex (depending on whether the method is in accordance with the third or fourth aspect of the invention) stimulation. In this embodiment, the timing control initiates the stimulation before the beginning of the pairing event, giving a negative delta t. A delay response timing system may initiate stimulation at the same time as the beginning of the pairing event, or after the beginning of the pairing event.

In another preferred embodiment of the third or fourth aspect of the invention, the electromagnetic coil is configured to deliver a single pulse of transcranial magnetic stimulation (TMS) to the hippocampus or to the motor cortex (depending on whether the method is in accordance with the third or fourth aspect of the invention) of the subject during the stimulation period. Preferably, said single pulse of TMS is apply at an intensity in the range of from about 80% to 160% of the Resting Motor Threshold (RMT) of the foot motor cortex to the hippocampus, wherein the RMT is the stimulation intensity that evokes 5 out of 10 times a Motor Evoked Potential (MEP) with a peak-to-peak amplitude of 50µV while the muscle is at a resting state. More preferably, said single pulse of TMS is apply at an intensity in the range of from about 100% to 140% of the Resting Motor Threshold (RMT) of the foot motor cortex to the hippocampus. Still more preferably, said single pulse of TMS is apply at an intensity of about 120% of the Resting Motor Threshold (RMT) of the foot motor cortex to the hippocampus.

In another preferred embodiment of the second aspect of the invention or of any of its preferred embodiments, the method further comprises means, such as an optical position sensor, to assist the coil placement in the patient scalp to direct the magnetic stimulation to the hippocampus.

In another preferred embodiment of the second aspect of the invention or of any of its preferred embodiments, the training actions are memory tasks to be performed by the patient, having a coding phase, a retention phase and a recovery phase.

In another preferred embodiment of the third or fourth aspect of the invention or of any of its preferred embodiments, the method comprises a processor unit which is configured to in addition to at least one of the repeated training actions being temporally linked with the delivery of transcranial magnetic stimulation (TMS) pulses to the hippocampus or to the motor cortex (depending on whether the method is in accordance with the third or fourth aspect of the invention)of the subject during a stimulation period, said training actions are repeated before the stimulation period without being temporally linked with the delivery of transcranial magnetic stimulation (TMS) pulses.

In another preferred embodiment of the third or fourth aspect of the invention or of any of its preferred embodiments, the method comprises a processor unit which is configured to in addition to at least one of the repeated training actions being temporally linked with the delivery of transcranial magnetic stimulation (TMS) pulses to the hippocampus or to the motor cortex (depending on whether the method is in accordance with the third or fourth aspect of the invention)of the subject during a stimulation period, said training actions are repeated one or more times after the stimulation period without being temporally linked with the delivery of transcranial magnetic stimulation (TMS) pulses.

In still another preferred embodiment of the third or fourth aspect of the invention or of any of its preferred embodiments, the method comprises a processor unit which is configured to repeatedly display at least four training actions, in particular a pre-assessment: Evaluation of the basal capacity of memorization of the patient through a learning or memory task, a PAS intervention: stimulation application temporally linking the delivery of a single pulse of TMS towards the hippocampus with endogenous activation of the hippocampal pathway due to the completion of the task, Post-assessment: Evaluation of the ability to memorize by performing the same task immediately after the intervention, and an optional second Post-assessment: evaluation of the memorization capacity 1, 2, 3, 4, 5, 10, 20 or preferably 30 minutes after the intervention.

It is noted that the plasticity induced by the systems or methods of the present invention can be further paired with a variety of therapies, rehabilitation, training and other forms of personal improvement. Each therapy acts as a training source. The specific timing requirements associated with each therapy are derived from the specifics of the therapy, such that the stimulation occurs during the training, and most effectively near the beginning of the training. Some possible therapies may include behavioral therapies such as sensory discrimination for sensory deficits, motor training for motor deficits, with or without robotic assistance and cognitive training/rehabilitation for cognitive deficits. Exercise and motor therapy could be paired to treat motor deficits arising from traumatic brain injury, stroke or Alzheimer's disease and movement disorders. Constraint induced therapy could be paired to help prevent the use of alternative strategies in order to force use of impaired methods. Speech therapy could be paired for speech and language deficits. Cognitive therapies could be paired for cognitive problems.

Multiple therapies could be paired simultaneously or sequentially.

Preferably the systems or methods of the present invention are for use in a method of treatment of a subject in need to improve at least one of concentration, memory, or cognitive performance. In particular, the systems or methods of the present invention are for use in a method of treatment of a subject in need to improve at least one of concentration, memory, or cognitive performance, wherein the subject is preferably diagnosed with ADHD (Attention deficit hyperactivity disorder) or suffering from age-related memory loss or at risk for age-related memory loss or suffering from any cognitive disorders including Dementia, Developmental disorders, Motor skill disorders, Amnesia, Substance-induced cognitive impairment, Alzheimer's disease, Behavioral variant frontotemporal dementia. Corticobasal degeneration, Huntington's disease, Lewy body dementia (or dementia with Lewy bodies), Mild cognitive impairment, Primary progressive aphasia, or Progressive supranuclear palsy. The subject may have significant difficulty focusing or concentrating. The subject may have developmental impairment of executive function. The subject may need treatment to improve cognitive performance in areas including but not limited to, standardized or other educational tasks or testing or occupational tasks or testing.

In addition, a fifth aspect of the invention refers to a neural stimulator as defined in the first or second aspect of the invention and means adapted to execute the steps of the said method.

A sixth aspect of the invention refers to a computer program [product] comprising instructions to cause the neural stimulator as defined in any aspect of the invention to execute the steps of the said method.

A seventh aspect of the invention refers to a computer-readable medium having stored there on the computer program of the sixth aspect.

None of the description in the present application should be read as implying that any particular element, step, or function is an essential element which must be included in the claim scope. The claims as filed are intended to be as comprehensive as possible, and NO subject matter is intentionally relinquished, dedicated, or abandoned.

The following examples are merely illustrative of the present invention but do not limit the same.

### Examples

### Example 1. Magnetic Stimulation of Human hippocampus during Memory Task.

### Materials and Methods

### 1. Previous analysis of human hippocampus depth in MRI images:

Eight anonymous MRI single-subject images of the entire brain of healthy subjects and a universal Montreal Neurological Institute (MNI) atlas were utilized for this study. MNI152 standard-space T1-weighted average structural template image utilized is derived from 152 structural images, averaged together after high-dimensional nonlinear registration into the common MNI152 co-ordinate system (MNl152_T1_1mm) (Collins, D. L., Zijdenbos, A. P., Kollokian, V., Sled, J. G., Kabani, N. J., Holmes, C. J., & Evans, A. C. (1998). Design and construction of a realistic digital brain phantom. IEEE transactions on medical imaging, 17(3), 463-468). Through the Statistical Parametric Mapping (SPM) package for Matlab (Friston et al., 1995; Ashburner and Friston, 1999), the images were normalized, taking the MNI152 atlas as a reference image.

Subsequently, with the aid of MRIcro software the coordinates of key locations were registered in each single subject MRI and in MNI152: most distal and proximal points of hippocampus, temporal surface, deepest foot cortex (toe cortex; Penfield W. & Rasmussen T., 1950), and frontal surface **(****Figure 1****).** Every key location was register in right and left hemisphere. The key locations were selected in a coronal view at the hippocampal-head level. The foot cortex locations were selected based on functional MRI (fMRI) studies (Weiss, C., et al., 2013) and the Haines neuroanatomy atlas (Haines, D. E., 2004). Then, the right and left hippocampi width, hippocampi middle point depth and foot cortex depth were calculated.

The **hippocampus width** was calculated in the coronal view for both hemispheres subtracting the absolute value of the mean of the x-axis of the proximal point to the mean of the x-axis of the distal point of the same hippocampus. This measurement is important to establish the deviation in millimeters that covers the hippocampus for the foot motor cortex comparison.

The **hippocampus middle point** was calculated in both hemispheres dividing the hippocampus width in half and adding the mean of the x-axis of the hippocampus proximal point. Then, the **hippocampus middle point depth** was calculated subtracting the absolute value of the middle point to the absolute value of the mean of the x-axis of the temporal surface key point.

**The foot cortex depth** was calculated in the sagittal view for both hemispheres, subtracting the absolute value of the mean z-axis foot motor cortex key point to the absolute value of the mean z-axis of the frontal surface key point.

The middle point of the hippocampus depth was compared with that of the foot motor cortex in individual RMIs, and then that distance was compared with the same comparison of the MNI252 atlas in order to know if the depth of the motor cortex falls within the range of the depth of the hippocampus width.

### 2. Magnetic Stimulation of Human hippocampus during Memory Task:

**Subjects.** 41 healthy volunteers (Age 21-43; 17 female) participated in this study. None of the subjects had past or present neurological disorders and they were not having any pharmacological treatment on the experiment day. All participants gave informed consent before the experimental intervention. The "Consejo Superior de Investigaciones Científicas" (CSIC) Ethics Committee approved all procedures.

**Experimental design:** The experimental phases of this study are the following: 1) **Measurement of the intensity** to be applied in the intervention. **2) Pre-assessment:** Evaluation of the basal capacity of memorization of the subject through a learning task. **3) PAS intervention:** stimulation application pairing a single pulse of TMS towards the hippocampus with endogenous activation of the hippocampal pathway due to the completion of the task. **4) Post-assessment:** Evaluation of the ability to memorize by performing the same task immediately after the intervention **5) Post-30'-assessment:** evaluation of the memorization capacity 30 minutes after the intervention.

### • Intensity determination

Based on the depth comparison described above, it is established as adequate intensity to apply 120% of the Resting Motor Threshold (RMT) of the foot motor cortex to direct the TMS pulse towards the hippocampus. The RMT is the stimulation intensity that evokes 5 out of 10 times a Motor Evoked Potential (MEP) with a peak-to-peak amplitude of 50µV while the muscle is at a resting state (Groppa, S., et al., 2012). Thus, the experimental session began with the subjects seated in a chair and their dominant foot was placed comfortably in a pillow wrapped in paper (hygienic issues).

Firstly, the point in the head where the foot stimulation (Flexor Hallucis Brevis muscle) is optimal (hot-spot) was found. We started with a TMS pulse of 50% of the maximum stimulator output until we found the RMT.

### • The assessment phases

Before, immediately after and 30 minutes after intervention (Pre-, Post- and Post-30'-assessment, respectively) a memory task is performed by the subject. The memory tasks are usually composed of a **coding phase,** in which the stimuli are presented, and the subject must try to memorize them; a **retention phase,** in which the elements to be memorized disappear, but the subject must retain; and finally, a **recovery phase,** where the subject indicates whether or not remembers them.

### Memory task

The memory task that the subject visualized in our study had a series of characteristics. The result must reflect short-term memory, it should be as simple as possible to rule out external factors, we did not want the items to be *Letters,* so that the person uses mnemonic rules to memorize as little as possible. Moreover, it was essential each cycle to have a coding phase and a recall phase, in order to apply the TMS in each trial. We wanted the memory performance to be not only recognition, but an exercise of active memory, to be able to give results limited to the capacity of memorization and not of recognition.

Given these characteristics, we adapted the "masking task" model task, presented by Sana Inoue and Tetsuro Matsuzawa, 2007. This task has a coding phase in which numbers are presented in a random arrangement within a button matrix; a retention phase, where the elements disappear, but leave a blank track; and a recovery phase in which the subject has to press the buttons following the numerical order of the items.

### • TMS-PAS Intervention Phase:

19 healthy volunteers (Age 21-31; 8 female) participated in this experimental group, which received the TMS-PAS intervention directed to hippocampal formation (named Hippocampus group).

### Neuronavigation

A frameless stereotactic Brainsight TMS Navigation assisted the coil placement in the subject scalp in order to direct the magnetic stimulation to hippocampal formation. It is based on an optical (infrared) position sensor NDI Polaris camera (Vicra / Spectra) tracking method. We aligned the participant's head and TMS coil position in 3D real space to MNI152 image space in the screen. Firstly, we calibrated the central point of the coil that will be in contact with the scalp with the trackers placed in the handle of the coil. Thus, we can identify the TMS central point in the screen space. Then, we determined 40 anatomical landmarks in the MRI image **(****Figure 2****)** that we identify in subject head (physical space coordinates), locating 3 trackers on a tape that is placed around the subject's head and using a tracked pointer to indicate physically the designated landmarks in subject head and face. This way, we could visualize in real time from physical space to image space, the center point of TMS coil location in relation to the head and brain structures, enabling the precise placement in the scalp to direct the stimulation to hippocampus.

### TMS-PAS Application:

The TMS PAS intervention is based in the convergence of two stimuli in the same neuronal population. We converged the TMS single pulse with the genuine activation of hippocampal formation in the medial temporal lobe of the brain by memorization task. Thus, we applied the TMS single pulse synchronized with the encoding phase of the memorization task, when the items to memorize are presented. There were two intervention phase and 25 trials of the memorization task paired with TMS in each phase (50 TMS single pulse). The TMS was applied at the 120% of Foot Motor Cortex RMT.

### Control groups:

### Task group

22 healthy volunteers (Age 21-43; 9 female) participated in this experimental group. At the intervention time, the subject performed the memory task without receiving any TMS. We designed this control group to assess the basal capacity of memorization restricted to the task training.

Statistical **Analysis:** The successfully remembered items in the memorization task rated the memorization capability. We collected the information through the program that executes the task and normalized the data based on the pre-assessment time, as the baseline. We calculated the Student T test in order to compare each experimental time (First Intervention, Second Intervention, Post-assessment and Post-30'-assessment) between groups (Hippocampus group and Task group).

The statistical procedures were calculated with the aid of statistical software IBM SPSS for every statistical analysis.

### Results

### 1. Previous analysis of human hippocampus depth in MRI images:

Based on the selected key points, right and left hippocampi width, hippocampi middle point depth and foot cortex depth were calculated (see **Table 1 below).**

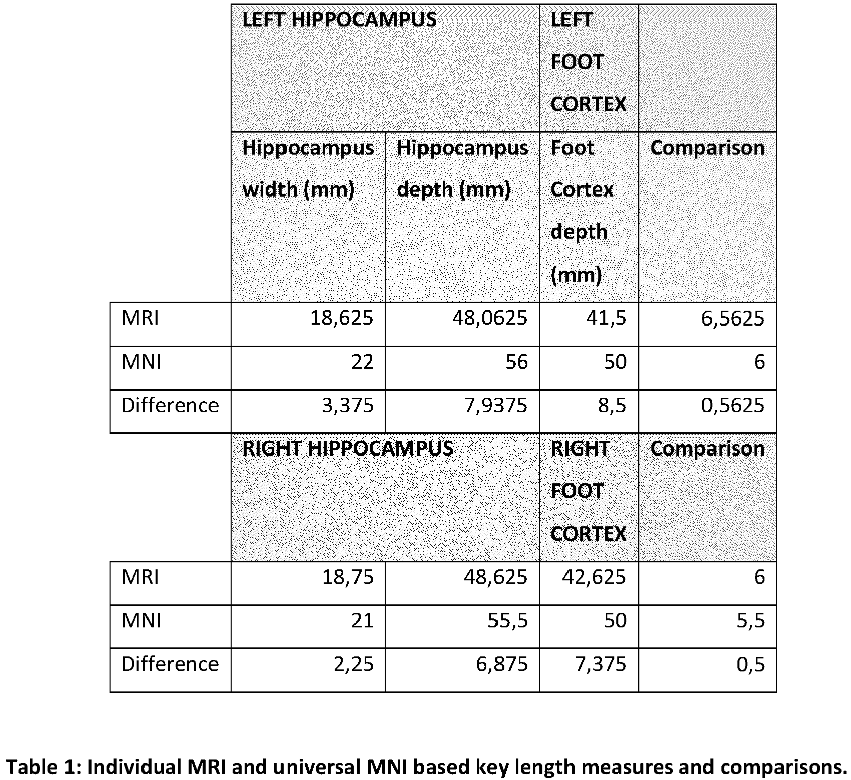

On one hand, the **midpoint of the left and the right hippocampi** for individual RMIs are located at a depth of 48mm and 48,6mm respectively, with a difference respect to the MNI152 of 7,9-6,8mm, being deeper in MNI152. On the other hand, **the depth of foot motor cortex** in left and right hemispheres for individual RMIs are located at 41,5mm and 42,6mm respectively and for universal MNI152 those are located at 50mm, resulting in a difference of 8,5-7,3mm and being deeper in MNI atlas. The differences between hippocampal middle point depth and foot motor cortex depth are the following: for the left hemisphere, 6,5mm in MRI and 6mm in MNI; for the right hemisphere, 6mm in MRI and 5,5mm in MNI.

Although there is a difference between depths of 6,5 to 5,5mm, the width of human hippocampus is of 18,6 to 22mm. This means that the hippocampus extends from its middle point to a minimum of 9.3 mm further on both sides, which implies that the depth of the foot motor cortex falls within the range of the hippocampus. In this way, we estimate that the intensity that triggers the foot motor cortex activation will also activate the hippocampal nervous cells when applying the TMS pulse towards that location.

### 2. Magnetic Stimulation of Human hippocampus during Memory Task:

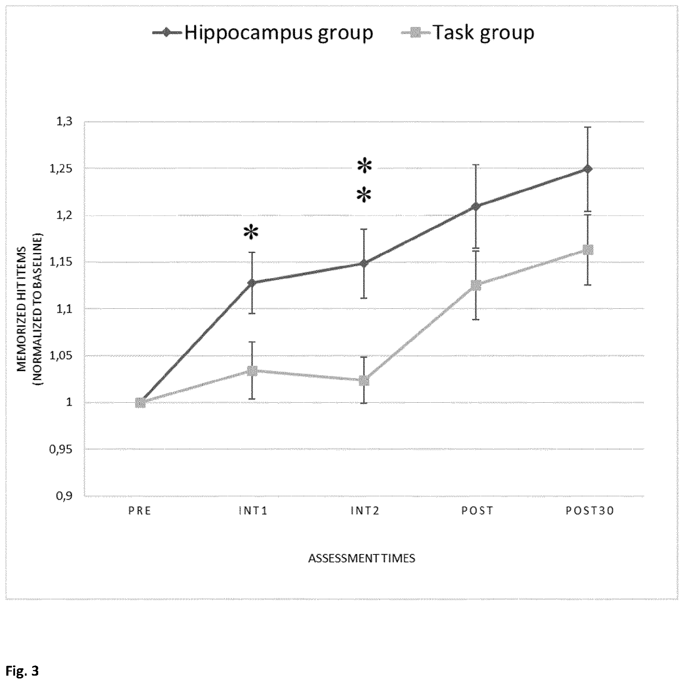

Comparing performance hit items between groups by Student T test, we demonstrated the potentiation effects on the memorization ability only due to our experimental design intervention in the PAS intervention times **(****Figure 3****).**

The subjects who were Stimulated by PAS protocol during the experimental process (Hippocampus group) demonstrated a higher memorization hit rate in intervention time 1 (Mean=1.128; SE=0.032) and intervention time 2 (Mean=1.148; SE=0.037) than the subjects who performed the task without PAS stimulation in intervention 1 (Mean= 1.034; SE=0.03; t(39)=2.112 , p=0.041, r=0.32) and intervention 2 (Mean=1.023; SE=0.025; t(32.16)= 2.811, p=0.008, r=0.444).

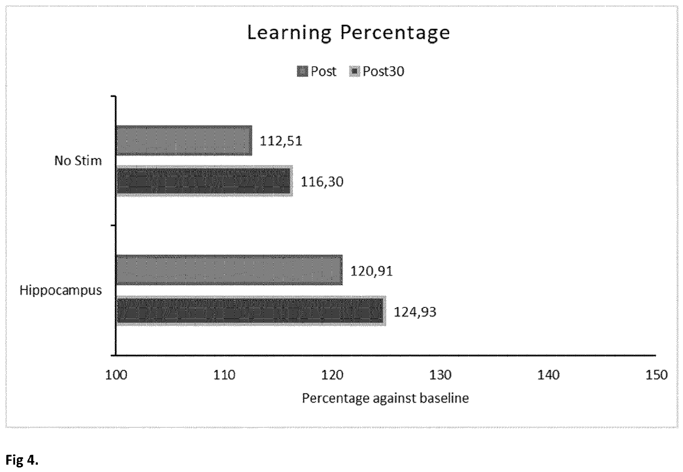

The subjects in the intervention directed to hippocampus group enhanced the most their memorization rate, in short- and long-term assessments, increasing its capacity up to 21% in the post-assessment and up to 25% in the post30-assessment. In the group without any stimulation, the task practice facilitated the memorization capacity a 12% in post-assessment and a 16% in post30-assessment. Thus, we can result that the TMS-PAS intervention directed to hippocampus boost the memorization cognition a 9% more than in a regular process of memorization **(****Figure 4****).**

### Example 2. Magnetic Stimulation of Human Motor Cortex during Memory Task.

### Materials and Methods

**Subjects.** 38 healthy volunteers (Age 21-38; 20 female) participated in this study. None of the subjects had past or present neurological disorders and they were not having any pharmacological treatment on the experiment day. All participants gave informed consent before the experimental intervention. The "Consejo Superior de Investigaciones Cientificas" (CSIC) Ethics Committee approved all procedures.

**Experimental design:** The experimental phases of this study are the following: **1) Measurement of the intensity** to be applied in the intervention. **2) Pre-assessment:** Evaluation of the basal capacity of memorization of the subject through a learning task. **3) PAS intervention:** stimulation application pairing a single pulse of TMS towards the hippocampus with endogenous activation of the hippocampal pathway due to the completion of the task. **4) Post-assessment:** Evaluation of the ability to memorize by performing the same task immediately after the intervention **5) Post-30'-assessment:** evaluation of the memorization capacity 30 minutes after the intervention.

### • Intensity determination

It was established as adequate intensity to apply 120% of the Resting Motor Threshold (RMT) of the foot motor cortex. The RMT is the stimulation intensity that evokes 5 out of 10 times a Motor Evoked Potential (MEP) with a peak-to-peak amplitude of 50µV while the muscle is at a resting state (Groppa, S., et al., 2012). Thus, the experimental session began with the subjects seated in a chair and their dominant foot was placed comfortably in a pillow wrapped in paper (hygienic issues).

Firstly, the point in the head where the foot stimulation (Flexor Hallucis Brevis muscle) is optimal (hot-spot) was found. We started with a TMS pulse of 50% of the maximum stimulator output until we found the RMT.

### • The assessment phases

Before, immediately after and 30 minutes after intervention (Pre-, Post- and Post-30'-assessment, respectively) a memory task is performed by the subject. The memory tasks are usually composed of **a coding phase,** in which the stimuli are presented, and the subject must try to memorize them; a **retention phase,** in which the elements to be memorized disappear, but the subject must retain; and finally, a **recovery phase,** where the subject indicates whether or not remembers them.

### Memory task

The memory task that the subject visualized in our study had a series of characteristics. The result must reflect short-term memory, it should be as simple as possible to rule out external factors, we did not want the items to be *Letters,* so that the person uses mnemonic rules to memorize as little as possible. Moreover, it was essential each cycle to have a coding phase and a recall phase, in order to apply the TMS in each trial. We wanted the memory performance to be not only recognition, but an exercise of active memory, to be able to give results limited to the capacity of memorization and not of recognition.

Given these characteristics, we adapted the "masking task" model task, presented by Sana Inoue and Tetsuro Matsuzawa, 2007. This task has a coding phase in which numbers are presented in a random arrangement within a button matrix; a retention phase, where the elements disappear, but leave a blank track; and a recovery phase in which the subject has to press the buttons following the numerical order of the items.

### • TMS-PAS Intervention Phase:

19 healthy volunteers (Age 21-38; 12 female) participated in this experimental group, which received the TMS-PAS intervention directed to foot motor cortex (named Motor Cortex group).

### TMS-PAS Application:

The TMS PAS intervention is based in the convergence of two stimuli in the same neuronal population. We converged the TMS single pulse with the genuine activation of motor cortex in the frontal lobe of the brain by memorization task (task needs movement and special memory to complete it). Thus, we applied the TMS single pulse synchronized with the encoding phase of the memorization task, when the items to memorize are presented. There were two intervention phase and 25 trials of the memorization task paired with TMS in each phase (50 TMS single pulse). The TMS was applied at the 120% of Foot Motor Cortex RMT.

### Control groups:

### Task group

22 healthy volunteers (Age 21-43; 9 female) participated in this experimental group. At the intervention time, the subject performed the memory task without receiving any TMS. We designed this control group to assess the basal capacity of memorization restricted to the task training.

### Statistical Analysis:

The successfully remembered items in the memorization task rated the memorization capability. We collected the information through the program that executes the task and normalized the data based on the pre-assessment time, as the baseline.

First, we calculated an ANOVA with intra-subject factor of TIME and inter-subject factor of Basal Efficiency (basal level of the first assessment in two levels; low efficiency and high efficiency) and Group factor (Motor Cortex group and Task group). We decided to performe this calculation due to the enhancement differences that we show between subject with low and high basal response.

We calculated the Student T test in order to compare each experimental time (First Intervention, Second Intervention, Post-assessment and Post-30'-assessment) between groups (Motor Cortex group and Task group) in low basal efficacy subjects which were benefited significantly more from PAS protocol.

The statistical procedures were calculated with the aid of statistical software IBM SPSS for every statistical analysis.

### Results

### 1. Magnetic Stimulation of Human motor cortex during Memory Task:

Due to the difference between low and high basal performance increment in memory, we calculated a repeated measure ANOVA with intra-subject factor of Time (intervention1, intervention2, post and post30 assessment) and inter-subject factor of Basal Efficacy (low and high) and Group (Motor Cortex and Task group).

The ANOVA resulted in a significant principal effect of Time in memorization enhancement F(3,111)= 13.663, p=0.000; a significant principal effect of Group F(1,37)=6,761, p=0.013; and a significant principal effect of Basal Efficacy F(1,37)=27,456, p=0.000. Moreover, there was a significant interaction between Group and Basal effect F(1,37), p=0,013.

At the post-hoc analysis, the Motor Cortex group (Mean= 1.210, SE=0.032), compared to Task group (Mean= 1.086, SE=0.035) significantly enhanced their memorization capability (p=0.013). Comparing low and high basal efficacy, only in low basal efficacy level, Motor Cortex group (Mean=1.395, SE=0.05) was significantly improving the memorization rate comparing to task group (Mean= 1.149, SE=0.046; p=0.001). Comparing the Basal Efficacy, only in motor cortex group, low efficacy level (Mean=1.395, SE=0.05) significantly enhanced memorization rate compared to high efficacy basal level (Mean=1.024, SE=0.048; p=0.000).

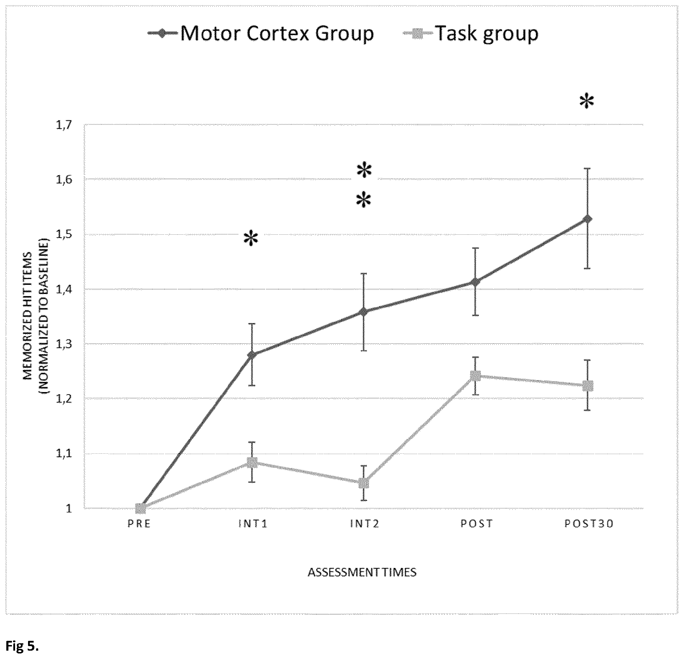

Due to these results we compared hit items between low basal efficiency groups by Student T test calculation, we demonstrated the potentiation effects on the memorization ability due to the PAS application to motor cortex in human brain in the PAS intervention times and post-30 minutes assessment (long term performance) **(****Figure 5****).**

The subjects who were stimulated by PAS protocol during the experimental process (Motor Cortex group) demonstrated a higher memorization hit rate in intervention time 1 (Mean=1.280; SE=0.082), intervention time 2 (Mean=1.358; SE=0.103) and Post30-assessment time (Mean=1.529; SE=0.133) than the subjects who performed the task without PAS stimulation in intervention 1 (Mean= 1.065; SE=0.047; t(17)=2.318, p=0.033, r=0.49) , intervention 2 (Mean=1.023; SE=0.039; t(17)= 3.177, p=0.006, r=0.61) and post30 assessment time (Mean=1.023; SE=0.039; t(17)= 2.201, p=0.042, r=0.47).

The subjects in the PAS directed to foot motor cortex enhanced the most their memorization rate, in short- and long-term assessments, increasing its capacity up to 41% in the post-assessment and up to 53% in the post30-assessment (more than half of the baseline performance of the subjects).

In the group without any stimulation, the task practice facilitated in low basal efficacy group a 24% of memorization capacity in post-assessment and a 22% in post30-assessment (the subjects do not increase their capacity in long term assessment).

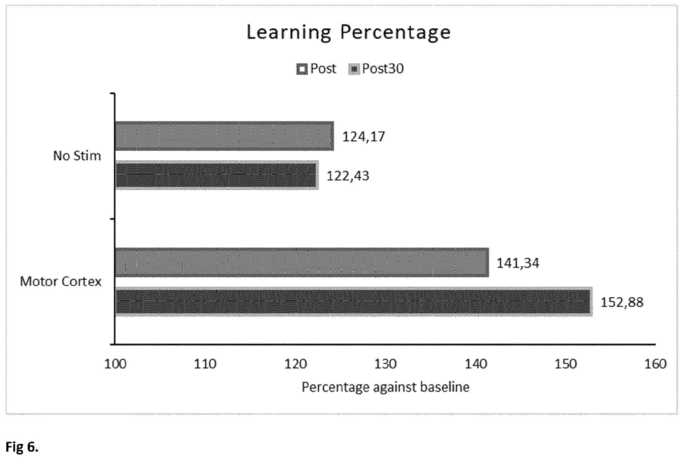

Thus, we can result that the TMS-PAS intervention directed to foot motor cortex boost the memorization cognition a 17% in short-term and 31% in long term more than in a regular process of memorization **(****Figure 6****).**

## Claims

1. A system for transcranial magnetic stimulation (TMS) configured to trigger the activation of the cortico-hippocampal memory system in a subject, wherein the system comprises:
a) an electromagnetic coil that receives power from a power source to produce a magnetic field, wherein the electromagnetic coil is configured to produce a magnetic field suitable for transcranial magnetic stimulation;
b) a processor unit that controls operation of the electromagnetic coil; and
c) optionally, an interface that includes the electromagnetic coil, the interface configured to position the electromagnetic coil to deliver controlled transcranial magnetic stimulation (TMS) pulses to the hippocampus of the patient during a stimulation period,
wherein the processor unit that controls operation of the electromagnetic coil is further configured to provide instructions to a subject connected to the system through a terminal that displays training actions such as memory tasks to the subject, according to an operating protocol, wherein said operating protocol is configured to repeatedly, randomly or subsequently, performing one or more training actions such as memory tasks, wherein at least one of the repeated performances is temporally linked with the delivery of transcranial magnetic stimulation (TMS) pulses to the hippocampus of the subject during a stimulation period.

2. The system according to claim 1, wherein the system comprises means, such as an optical position sensor or a Neuronavigator, to assist the coil placement in the subject scalp in order to direct the magnetic stimulation to the hippocampal formation, and wherein the system optionally further comprises a plurality of electrodes configured to receive a signal indicative of brain activity in the subject and to provide the electrical signal to the processor unit.

3. The system of claim 1 or 2, wherein the processor unit is configured so that the electromagnetic coil delivers a single pulse of transcranial magnetic stimulation (TMS) to the hippocampus of the patient during the stimulation period.

4. .The system of claim 3, wherein said single pulse of TMS is apply at an intensity in the range of from about 80% to 160% of the Resting Motor Threshold (RMT) of the foot motor cortex to the hippocampus, wherein the RMT is the stimulation intensity that evokes 5 out of 10 times a Motor Evoked Potential (MEP) with a peak-to-peak amplitude of 50µV while the muscle is at a resting state.

5. The system of claim 3, wherein said single pulse of TMS is apply at an intensity in the range of from about 100% to 140% of the Resting Motor Threshold (RMT) of the foot motor cortex to the hippocampus.

6. The system of claim 3, wherein said single pulse of TMS is apply at an intensity of about 120% of the Resting Motor Threshold (RMT) of the foot motor cortex to the hippocampus.

7. The system according to any of claims 1 to 6, wherein the training actions are memory tasks to be performed by the subject, having a coding phase, a retention phase and a recovery phase.

8. The system according to any of claims 1 to 7, wherein the processor unit is configured to in addition to at least one of the repeated training actions being temporally linked with the delivery of transcranial magnetic stimulation (TMS) pulses to the hippocampus of the subject during a stimulation period, said training actions are repeated before the stimulation period without being temporally linked with the delivery of transcranial magnetic stimulation (TMS) pulses.

9. The system according to any of claims 1 to 8, wherein the processor unit is configured to in addition to at least one of the repeated training actions being temporally linked with the delivery of transcranial magnetic stimulation (TMS) pulses to the hippocampus of the subject during a stimulation period, said training actions are repeated one or more times after the stimulation period without being temporally linked with the delivery of transcranial magnetic stimulation (TMS) pulses.

10. The system according to any of claims 1 to 9, wherein the processor unit is configured to repeatedly display at least four training actions, in particular a pre-assessment: Evaluation of the basal capacity of memorization of the subject through a learning or memory task, a PAS intervention: stimulation application temporally linking the delivery of a single pulse of TMS towards the hippocampus with endogenous activation of the hippocampal pathway due to the completion of the task, Post-assessment: Evaluation of the ability to memorize by performing the same task immediately after the intervention, and an optional second Post-assessment: evaluation of the memorization capacity 1, 2, 3, 4, 5, 10, 20 or preferably 30 minutes after the PAS intervention.

11. A method carried out by a computer to trigger the activation of the cortico-hippocampal memory system in a subject in need thereof by using a neural stimulator comprising:
a) an electromagnetic coil that receives power from a power source to produce a magnetic field, wherein the electromagnetic coil is configured to produce a magnetic field suitable for transcranial magnetic stimulation;
b) a processor unit that controls operation of the electromagnetic coil; and
c) an interface that includes the electromagnetic coil, the interface configured to position the electromagnetic coil to deliver controlled transcranial magnetic stimulation (TMS) pulses to the hippocampus of the patient during a stimulation period,
wherein the processor unit that controls operation of the electromagnetic coil is further configured to provide instructions to a subject connected to the neural stimulator through a terminal that displays training actions such as memory tasks to the subject, according to an operating protocol, wherein the method comprises, in accordance with the operating protocol, to repeatedly performing one or more training actions such as memory tasks, wherein at least one of the repeated performances is temporally linked with the delivery of transcranial magnetic stimulation (TMS) pulses to the hippocampus of the subject during a stimulation period.

12. The method of claim 11, wherein the electromagnetic coil delivers a single pulse of transcranial magnetic stimulation (TMS) to the hippocampus of the patient during the stimulation period.

13. The method of claim 12, wherein said single pulse of TMS is apply at an intensity in the range of from about 80% to 160% of the Resting Motor Threshold (RMT) of the foot motor cortex to the hippocampus, wherein the RMT is the stimulation intensity that evokes 5 out of 10 times a Motor Evoked Potential (MEP) with a peak-to-peak amplitude of 50µV while the muscle is at a resting state.

14. The method of claim 12, wherein said single pulse of TMS is apply at an intensity in the range of from about 100% to 140% of the Resting Motor Threshold (RMT) of the foot motor cortex to the hippocampus.

15. The method of claim 12, wherein said single pulse of TMS is apply at an intensity of about 120% of the Resting Motor Threshold (RMT) of the foot motor cortex to the hippocampus.

16. The method according to any of claims 11 to 15, wherein the neural stimulator further comprises means, such as an optical position sensor or a Neuronavigator, to assist the coil placement in the patient scalp to direct the magnetic stimulation to the hippocampus.

17. The method according to any of claims 11 to 16, wherein the training actions are memory tasks to be performed by the patient, having a coding phase, a retention phase and a recovery phase.

18. The method according to any of claims 11 to 17, wherein in addition to at least one of the repeated training actions being temporally linked with the delivery of transcranial magnetic stimulation (TMS) pulses to the hippocampus of the subject during a stimulation period, said training actions are repeated before the stimulation period without being temporally linked with the delivery of transcranial magnetic stimulation (TMS) pulses.

19. The method according to any of claims 11 to 18, wherein in addition to at least one of the repeated training actions being temporally linked with the delivery of transcranial magnetic stimulation (TMS) pulses to the hippocampus of the subject during a stimulation period, said training actions are repeated one or more times after the stimulation period without being temporally linked with the delivery of transcranial magnetic stimulation (TMS) pulses.

20. The method according to any of claims 11 to 19, wherein the method displays at least four training actions, in particular a pre-assessment: Evaluation of the basal capacity of memorization of the subject through a learning or memory task, a PAS intervention: stimulation application temporally linking the delivery of a single pulse of TMS towards the hippocampus with endogenous activation of the hippocampal pathway due to the completion of the task, Post-assessment: Evaluation of the ability to memorize by performing the same task immediately after the intervention, and an optional second Post-assessment: evaluation of the memorization capacity 1, 2, 3, 4, 5, 10, 20 or preferably 30 minutes after the PAS intervention.

21. A neural stimulator as defined in any of claims 11 to 20 and means adapted to execute the steps of the methods of any of claims 11 to 20.

22. A computer program [product] comprising instructions to cause the neural stimulator as defined in any of claims 11 to 20 to execute the steps of the methods of any of claims 11 to 20.

23. A computer-readable medium having stored thereon the computer program of claim 22.
